(19) Europäisches Patentamt
European Patent Office
Office européen des brevets

(11) Publication number: **0 269 569 B1**

(12) **EUROPEAN PATENT SPECIFICATION**

(45) Date of publication of patent specification: **13.05.92**

(51) Int. Cl.[5]: **C07C 67/08**

(21) Application number: **87810674.9**

(22) Date of filing: **18.11.87**

(54) **Esterification process with calcium hydroxybenzyl phosphonate-phenol sulfide catalyst system.**

(30) Priority: **24.11.86 US 934292**

(43) Date of publication of application:
**01.06.88 Bulletin 88/22**

(45) Publication of the grant of the patent:
**13.05.92 Bulletin 92/20**

(84) Designated Contracting States:
**BE DE FR GB IT SE**

(56) References cited:
**EP-A- 0 021 838**
**US-A- 2 776 985**
**US-A- 4 659 514**

(73) Proprietor: **CIBA-GEIGY AG**
**Klybeckstrasse 141**
**CH-4002 Basel(CH)**

(72) Inventor: **Geib, Jay R.**
**6 Ledge Lane**
**Stamford Connecticut 06905(US)**
Inventor: **Windus, John J.**
**993 Lester Road**
**Yorktown New York 10598(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.08/2.19/2.0)

## Description

It is known that esterfication reactions can be conducted in the presence of a variety of esterification catalysts. However, numerous difficulties are encountered in rosin ester, polyester and polymeric ester esterifications utilizing the state of the art catalysts. These difficulties can be grouped into unduly prolonged esterification times, poor oxygen stability and color formation. More specifically, the prolonged reaction times required to complete the esterification reaction are uneconomical and, more importantly, may effect decarboxylation of the acid so as to eliminate sites for the esterification reaction with resulting incorrect reaction stoichiometry and incomplete reaction. Impure products result. The oxygen instability of the resulting esters is particularly in evidence during initial storage, there being a propensity to form hydroperoxides and peroxides, and during subsequent use thereby severely limiting the applicability of the esters. Uses as plasticizers in polymers and, for rosin esters, as tackifiers in adhesives are substantially foreclosed by such instability. Likewise, the formation of dark coloration substantially limits the areas of use for the resulting esters.

Frequently used acidic and basic esterification catalysts tend to promote the difficulties noted hereinabove. While various metal salts such as calcium formate, calcium stearate and calcium acetate provide satisfactory catalytic activity, they do not provide antioxidant or color inhibition effects. Accordingly, it is essential to post-add antioxidants and other stabilizers in order to obtain long-term stability. U.S. Patent No. 4,659,514 provides an improved system in disclosing the use of a calcium hydroxybenzyl phosphonate as a catalyst in such esterification reactions. Although significant improvements in catalytic activity and stabilization are obtained, color improvement and long term stabilization characteristics are still not present to a sufficient degree.

Reference is also made to U.S. 4,172,070 wherein rosin esters are prepared in the presence of arylsulfonic acid catalysts. Although improved esterfication and stability is indicated, longer reaction times than desired are still required, frequently poor color results and some decarboxylation occurs. The decarboxylation is a more serious problem when the acid is added to the initial reaction mix or during early stages of the esterification reaction. Such decarboxylation results in the formation of low molecular weight materials which must necessarily be removed in order to obtain esters of appropriate physical properties such as softening points. It is also required to wash the sulfonic acid out of the polyester in order to obtain purer products and to consider the possibility of some post-addition of antioxidants in view of the continuing proclivity to form hydroperoxides or peroxides during processing and storage.

In addition, various disproportionation techniques have been employed in an attempt to obtain lighter color, stabilized rosin esters. Such approaches have involved holding the reactants at elevated temperatures for a prescribed period of time and then distilling or heating in the presence of a sulfur compound such as SANTONOX® R (4,4′-thiobis(2-methyl-5-tert-butylphenol) or a variety of other sulfide compounds (see U.S. 3,377,333 and 3,377,334 among others). Similarly such sulfides have been taught to improve color in rosin-pentaerythritol and rosin-glycerol esters. Reference is made to U.S. 3,780,013 and U.S. 4,447,354 in this regard. Although such sulfides provide improved characteristics in that they reduce color, further improvement in color and particularly in color and oxygen stabilization during subsequent processing, storage and/or use in formulated systems is still required. Furthermore, these patents are generally silent on other previously noted disadvantages and methods for improvement, e.g. reaction rates.

It is, therefore, the primary object of this invention to provide an esterification catalyst system which provides rapid esterification reaction times so as to substantially eliminate the problems associated with prolonged reactions and which facilitates obtaining pure, completely esterified products exhibiting reduced color formation and subsequent oxygen and storage stability during use.

Other objects and advantages of this invention will become apparent from the following description thereof.

It has now been surprisingly discovered that by utilizing a combination of calcium bis[monoethyl(3,5-di-tert.butyl-4-hydroxybenzyl)phosphonate and a phenol sulfide as an esterification catalyst for rosin esters, esters of significantly improved performance characteristics are obtained. Thus, the reaction conditions minimize decarboxylation and result in much purer products. The need for special removal steps is substantially eliminated. Esters of desired softening points are prepared. Similar advantages are obtained in the preparation of other polyesters. The mixed catalyst system also provides esters having excellent antioxidant properties. Correspondingly, post-esterification addition of stabilizers is not required. The absence of formation of hydroperoxide and peroxide groups during storage and the long term stability of the polyesters and polyester-containing formulations are evidence of such characteristics. Color formation in the polyesters and formulated products is likewise substantially reduced. Thus, oxygen-stable esters of

good color and good purity are prepared. It is particularly in the area of reduced color and prolonged oxidation stability that the instant systems exhibit significant improvement over the prior art and over the performance of the individual components of the catalyst system.

The present invention relates, therefore, to an esterification procedure wherein an aliphatic or aromatic carboxylic acid or anhydride is reacted with an equivalent amount or a maximum 30% excess of an alcohol at elevated temperatures from 150 to 300 °C in the presence of a catalyst for a period of time sufficient to yield an ester having a maximum acid number of about 15, the improvement comprising conducting said reaction in the presence of from 0.05-10.0%, by weight, of a blend of calcium bis[monoethyl(3,5-di-tert.butyl-4-hydroxybenzyl)-phosphonate] and a phenol sulfide corresponding to the formula

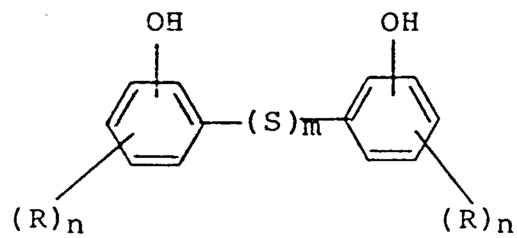

wherein R is $C_1$-$C_{18}$ alkyl, $C_5$-$C_{12}$ cycloalkyl, $C_7$-$C_9$ aralkyl or $C_7$-$C_9$ aralkyl substituted by $C_1$-$C_{12}$ alkyl; n is 1-3; and m is 1-3.

The process of this invention involves reacting aliphatic or aromatic carboxylic acids with appropriate alcohols at temperatures ranging from 150 to 300 °C.

A process is preferred, wherein said acid is an aliphatic $C_1$-$c_{18}$ monocarboxylic acid, aliphatic $C_2$-$C_{18}$ dicarboxylic acid, aliphatic tricarboxylic acid, aromatic mono-, di- or tri-carboxylic acid or rosin acid.

Typical aliphatic acids include $C_1$-$C_{18}$ mono-carboxylicacids such as butyric, caproic, lauric, myristic, palmitic, stearic, oleic, linoleic and linolinic acids; $C_2$-$C_{18}$ dicarboxylic acids such as succinic, glutaric, adipic, azelaic, sebacic and maleic acids; tricarboxylic acids such as citric acid; and rosin acids (abietic and pimaric acid types). Aromatic mono-, di- and tri-carboxylic acids include benzoic acid, phthalic acid, isophthalic acid, terephthalic acid, methoxy benzoic acid, naphthoic acid, cinnamic acid and trimellitic acid; as well as aralkyl and alkaryl carboxylic acids and anhydrides such as phthalic and trimellitic anhydrides. The rosin acids are preferred for use in this invention.

Further, a process is preferred, wherein said alcohol is a $C_1$-$C_{18}$ monohydric alcohol, $C_2$-$C_{18}$ diol or polyhydric alcohol, and especially pentaerythritol or glycerol.

Typical alcohols include $C_1$-$C_{18}$ monohydric alcohols such as ethanol, butanol, octanol, dodecanol and stearyl alcohol; $C_2$-$C_{18}$ diols such as ethylene glycol, propanediol, butanediol, butylene diol and hexanediol; polyhydric alcohols such as glycerol, pentaerythritol, dipentaerythritol, tripentaerythritol, trimethylolethane and trimethylolpropane; and naturally occurring sugars such as dextrose or sucrose. The polyhydric alcohols are preferred. Stoichiometric amounts of acid and alcohol are utilized, with the possibility of utilizing up to about a 30% equivalent excess of alcohol if a polyhydric alcohol is used, with a 5-30% excess particularly applicable. As noted, the invention is particularly applicable for preparing di-, tri- or tetra-esters as well as polymeric esters such as polyethylene terephthalate.

The calcium bis[monoethyl(3,5-di-tert.butyl-4-hydroxybenzyl) phosphonate] falls within the disclosure of U.S. 3,310,575. Preparative procedures and areas of use are noted in said patent. The phosphonate is commercially available.

The applicable phenol mono-, di- and tri- sulfides correspond to the formula

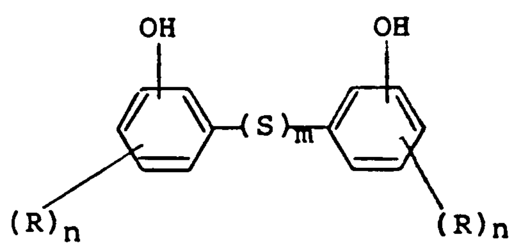

wherein R is $C_1$-$C_{18}$ alkyl, $C_5$-$C_{12}$ cycloalkyl, $C_7$-$C_9$ aralkyl or $C_7$-$C_9$ aralkyl substituted by $C_1$-$C_{12}$ alkyl;

n is 1-3; and

m is 1-3.

Preferred compounds within the above structure are those wherein the hydroxy group is ortho or para to the sulfide linkage, n is 2, m is 2 and the R groups are in each meta position to the sulfide linkage. The R groups are preferably straight-chain or branched alkyl with 1 to 12 carbon atoms, such as methyl, n-butyl, sec.butyl, tert.butyl, tert.pentyl, 2-ethylhexyl, n-octyl, 1,1,3,3-tetramethylbutyl, n-decyl and n-dodecyl. The groups methyl, tert.butyl, tert.pentyl and 1,1,3,3-tetramethylbutyl are especially preferred.

Cycloalkyl substituents include cyclopentyl and cyclohexyl, while aralkyl groups include benzyl, alphamethylbenzyl and alpha,alpha-dimethylbenzyl.

A representative number of phenol monosulfides are disclosed in U.S. 3,337,334 and the corresponding di- and trithiobis variants are suggested. Preferred disulfides for purposes of this invention include:

4,4′-dithiobis(2,6-di-tert.butylphenol)
4,4′-dithiobis(2-methyl-6-tert.butylphenol)
2,2′-dithiobis(4,6-di-tert.-butylphenol)
2,2′-dithiobis(4-methyl-6-tert.butylphenol)
2,2′-dithiobis(4,6-dimethylphenol)
4,4′ dithiobis(2,6-dimethylphenol)

It is also to be noted that such disulfides in admixture with minor amounts of the corresponding mono- and trivariants are likewise preferred and are intended as falling within the definition of disulfides for purposes of this invention. These compounds are commercially available or can be prepared by known methods.

The catalyst system is utilized in concentrations of from about 0.05-10.0%, by combined weight of acid and alcohol, preferably from about 0.2-1.5%, and most preferably about 0.5-1.0%. The individual phosphonate and sulfide components are present in weight ratios ranging from 10:1 to 1:20, and most preferably 2:1 to 1:1.

The catalyst system may be added prior to the initiation of the esterification reaction or at a designated point during the reaction when the original acid number has been reduced by about 10 to 67%. Completion of the reaction is determined by monitoring of the acid number, esters of acid number below about 15 generally being desired. The process of the invention will generally not exceed seven hours in order to prepare the desired esters. The resulting esters show excellent color and extended oxidation stability during storage and as part of formulated products. The esters are of particular value when used as tackifiers in various adhesive formulations such as ethylene/vinyl acetate hot melt adhesives.

The following examples will further illustrate the embodiments of the instant invention. In these examples, all parts and percentages are by weight unless otherwise noted.

## TEST COMPOUNDS

A - calcium bis[monoethyl-(3,5-di-tert.butyl-4-hydroxybenzyl)-phosphonate
B - 4,4′-dithiobis(2,6-di-tert.butylphenol)
C - 4,4′-dithiobis(2-methyl-6-tert.butylphenol)
D - 2,2′-dithiobis(4,6-di-tert.butylphenol)
E - mixed polysulfide of p-tert.amylphenol - VULTAC® 2
F - 2,2′-thiobis(4,6-di-tert.butylphenol)
G - 4,4′-thiobis(2,6-di-tert.butylphenol)
H - 4,4′-thiobis(2-methyl-5-tert.butylphenol)
I - 2,2′-thiobis(4,6-dimethylphenol)
J - 2,2′-thiobis(4-methyl-6-tert.butylphenol)
K - 2,2′-trithiobis(4-methyl-6-tert.butylphenol)
L - 2,2′-trithiobis(4,6-di-tert.butylphenol)

## EXAMPLE 1

A reaction vessel is charged with 500 grams tall oil rosin acid-acid number 179, 71.1 grams pentraerythritol (30% stoichiometric excess), the indicated amounts of catalyst components and 25 grams xylene and heated rapidly to 275 °C. Heating is continued at 275 °C and acid numbers are periodically determined. Heating is discontinued when an acid number in the area of 15 or less is obtained and the softening point (determined by the ring and ball method-ASTM E28-67) is in the 90-100 °C range. The latter softening point can be raised by continued heating with an increased nitrogen flow to remove impurities.

The values for the various prepared rosin acid esters are noted below.

| Ester | Components | Conc. (%, by wt.) | Reaction Time (hrs.) | Acid No. | Soft Pt.(°C) | Gardner Color |
|---|---|---|---|---|---|---|
| 1. | - | - | 8 | 35 | - | 13 |
| 2. | A | 0.25 | 7.75 | 15 | 99 | 12 |
| 3. | A | 0.50 | 7.2 | 16 | 97 | 11 |
| 4. | A | 1.0 | 6.75 | 11 | 102 | 10-11 |
| 5. | B | 0.50 | - | - | - | 8 |
| 6. | B | 1.0 | 15.0 | 20 | 92 | 8 |
| 7. | A/B | 0.5/0.5 | 7.2 | 16 | 94 | 6-7 |
| 8. | A/B | 0.5/0.25 | 7.0 | 13 | 100 | 6-7 |
| 9. | A/C | 0.5/0.5 | 6.6 | 13 | 99 | 7 |
| 10. | D | 1.0 | >8.5 | 36(8hr) | - | 8-9 |
| 11. | A/D | 0.5/0.5 | 7.4 | 17 | 87 | 7 |
| 12. | A/D | 0.5/0.25 | 6.6 | 14 | 97 | 6-7 |
| 13. | A/E | 0.5/0.5 | 7.2 | 17 | 83 | 6-7 |
| 14. | A/F | 0.5/0.5 | 7.2 | 16 | 90 | 7 |
| 15. | A/G | 0.5/0.5 | 6.6 | 16 | 97 | 8.5 |
| 16. | A/H | 0.5/0.5 | 7.0 | 13 | 97 | 6-7 |
| 17. | A/I | 0.5/0.5 | - | - | - | - |
| 18. | A/J | 0.5/0.5 | 7.25 | 15 | 94 | 8 |
| 19. | A/J | 0.5/0.25 | 6.6 | 15 | 98 | 8 |
| 20. | A/K | 0.5/0.5 | - | - | - | 5 |
| 21. | A/L | 0.5/0.5 | - | - | - | 6 |

The oxygen stability of the individual esters is determined by grinding the product to a 40 mesh size, aging under different conditions and determining hyproperoxide values by iodometric titration. Since a greater presence of hydroperoxides is indicative of less oxygen stability, lower hydroperoxide values are desired.

Differential scanning colorimetry (DSC) is also conducted on the esters determining the time to exotherm at 100°C for the unaged ester. This procedure is indicative of oxidative stability and is conducted by the method described in Thermochimica Acta 91, 87-94 (1985).

The results are noted below.

| Ester | Hydroperoxide Content (ppm) | | | | | | DSC (min) |
|---|---|---|---|---|---|---|---|
| | Weeks at Ambient Temp. | | | | Days at 50°C | | |
| | 0 | 3 | 6 | 9 | 5 | 10 | |
| 2 | 65 | 430 | 570 | 1600 | 8100 | 7600 | 60 |
| 3 | 55 | 610 | 590 | 1600 | 8100 | 9100 | 55 |
| 4 | 200 | 600 | - | - | 3400 | 7700 | - |
| 5 | 40 | - | 400 | 840 | - | - | - |
| 7 | 10 | 250 | 440 | 900 | 1700 | 3700 | 280 |
| 8 | 10 | - | - | - | 2600 | - | - |
| 9 | 10 | 190 | 235 | 1100 | 2400 | 3400 | 270 |
| 11 | 10 | 200 | 480 | 700 | 265 | 800 | 550 |
| 12 | 10 | - | - | - | 3200 | - | - |
| 13 | 10 | 190 | 310 | - | 650 | 2300 | 460 |
| 14 | 10 | 210 | 620 | 900 | 1800 | 4300 | 350 |
| 15 | 20 | 240 | 430 | 800 | 2800 | 4200 | 235 |
| 16 | 200 | - | - | - | 2300 | 4200 | - |
| 17 | 20 | - | 425 | 690 | - | - | - |

These data thus indicate both the excellent color and oxidative stability of the system of this invention.

Example 2

This example illustrates the oxidative stability of the instant systems in end use applications. Thus, the various esters, either unaged or after having been aged at 50°C for 5 and 10 days respectively, are incorporated into the following hot melt adhesive:

| | parts |
|---|---|
| ethylene/vinyl acetate (ELVAX® 250) | 1 |
| microcrystalline wax | 1 |
| rosin ester | 1 |

The resulting adhesives are then aged in an open beaker in a forced air oven maintained at 177°C. Samples are analyzed periodically to determine Gardner Color and viscosity (determined on a Brookfield Thermosel System using a No. 21 spindle at 10 rotations per minute at 177°C). The following results are obtained.

| Adhesive Initial Properties | | | | | | |
|---|---|---|---|---|---|---|
| Rosin Ester Tackifier Aged 0, 5 and 10 days at 50°C | | | | | | |
| Ester | Gardner Color | | | Viscosity (cP) | | |
| | 0 | 5 | 10 | 0 | 5 | 10 |
| 2 | 6 | 10 | 11 | 2850 | 3150 | 3625 |
| 3 | 6 | 10 | 11 | 2825 | 2950 | 3650 |
| 4 | 5 | 7 | 8 | 3000 | 2925 | 3400 |
| 7 | 5 | 5 | 6 | 2725 | 2575 | 2650 |
| 8 | 3 | 4 | 7 | 2925 | 2650 | 2875 |
| 9 | 6 | 6 | 8 | 2850 | 2625 | 2775 |
| 11 | 5 | 6 | 6 | 2700 | 2375 | 2575 |
| 12 | 3 | 5 | 6 | 2300 | 2725 | 2900 |
| 13 | 5 | 5 | 6 | 2825 | 2575 | 2750 |

| Adhesive Properties - Aged at 177°C for 24 Hours | | | | | | |
|---|---|---|---|---|---|---|
| Rosin Ester Tackifier Aged 0, 5 and 10 days at 50°C | | | | | | |
| Ester | Gardner Color | | | Viscosity (cP) | | |
| | 0 | 5 | 10 | 0 | 5 | 10 |
| 2 | 8 | 12 | 14 | 2775(s) | 3150 | 4125 |
| 3 | 7 | 12 | 14 | 2650(s) | 2950 | 3900 |
| 4 | 8 | 9 | 11 | 2425(s) | 2925 | 3275 |
| 7 | 6 | 9 | 10 | 2675 | 2575 | 2650 |
| 11 | 6 | 8 | 9 | 2750 | 2375 | 2400 |
| 13 | 6 | - | 10 | 2675 | 2575 | 2875 |

(s) = skinning of adhesive indicative of degradation

| Adhesive Properties - Aged at 177°C for 48 Hours | | | | | | |
|---|---|---|---|---|---|---|
| Rosin Ester Tackifier Aged 0, 5 and 10 days at 50°C | | | | | | |
| Ester | Gardner Color | | | Viscosity (cP) | | |
| | 0 | 5 | 10 | 0 | 5 | 10 |
| 2 | 11 | 14 | 17 | 2750 | 3150 | 4125 |
| 3 | 10 | 13 | 16 | 2750 | 2950 | 3900 |
| 4 | 11 | 12 | 14 | 2625 | 2925 | 3275 |
| 7 | 11 | 11 | 13 | 2750 | 2575 | 2650 |
| 8 | 11 | 12 | 12 | 2550 | 2650 | 2925 |
| 9 | 12 | 13 | 14 | 2875 | 2625 | 2850 |
| 11 | 10 | 10 | 11 | 2800 | 2375 | 2400 |

These data thus illustrate the improvement in color and viscosity stability of adhesive formulations containing rosin esters prepared according to the invention. Furthermore, the preparation of ethylene/vinyl acetate hot melt adhesives using aged rosin esters generally results in adhesives with higher color and viscosity than those prepared with unaged rosin esters. In contrast, adhesives prepared from rosin esters of this invention exhibit the desired pattern of no significant change in initial properties when using aged rosin esters, this pattern being particularly meaningful and evident in the test data relating to the initial properties of the adhesive formulations.

Example 3

A reaction vessel is charged with 250 grams distilled tall oil rosin acid (acid number 185) and the indicated amounts of catalyst components and the rosin is melted. Thereafter, 28 grams of glycerol are added at 160°C. The mixture is then heated at 260°C for six hours and acid numbers are periodically determined. When the acid number reaches 50, 200 mbar of vacuum is applied. Heating is discontinued when an acid number in the area of 7-8 is obtained.

The values for the various prepared rosin acid esters are noted below.

| Ester | Components | Conc. (%, by wt.) | Reaction Time (hrs.) | Acid No. | Gardner Color |
|---|---|---|---|---|---|
| 22. | A | 0.5 | 8 | 7 | 7 |
| 23. | A/B | 0.5/0.5 | 8.45 | 8 | 4 |
| 24. | A/H | 0.5/0.5 | 8.45 | 8 | 5 |
| 25. | A/J | 0.5/0.5 | 8.45 | 7 | 4 |

Hydroperoxide contents are also determined on samples aged at 30°C. The results are noted below.

| Ester | Hydroperoxide Content (mMol/g) | |
|---|---|---|
| | 15 days at 30°C | 30 days at 30°C |
| 22 | 0.052 | 0.121 |
| 23 | 0.028 | 0.051 |
| 24 | 0.058 | 0.102 |
| 25 | 0.034 | 0.078 |

Summarizing, it is seen that this invention provides an improved esterification process. Variations may be made in proportions, procedures and materials without departing from the scope of the invention as defined by the following claims.

## Claims

**1.** In an esterification procedure wherein an aliphatic or aromatic carboxylic acid or anhydride is reacted with an equivalent amount or a maximum 30% excess of an alcohol at elevated temperatures from 150 to 300 °C in the presence of a catalyst for a period of time sufficient to yield an ester having a maximum acid number of about 15, the improvement comprising conducting said reaction in the presence of from 0.05-10.0%, by weight, of a blend of calcium bis[monoethyl(3,5-di-tert.butyl-4-hydroxybenzyl)-phosphonate] and a phenol sulfide corresponding to the formula

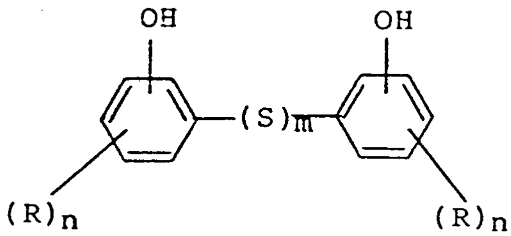

wherein R is $C_1$-$C_{18}$ alkyl, $C_5$-$C_{12}$ cycloalkyl, $C_7$-$C_9$ aralkyl or $C_7$-$C_9$ aralkyl substituted by $C_1$-$C_{12}$ alkyl; n is 1-3; and m is 1-3.

**2.** The process of claim 1, wherein said acid is an aliphatic $C_1$-$C_{18}$ monocarboxylic acid, aliphatic $C_2$-$C_{18}$ dicarboxylic acid, aliphatic tricarboxylic acid, aromatic mono-, di- or tri-carboxylic acid or rosin acid.

**3.** The process of claim 2, wherein said acid is a rosin acid.

**4.** The process of claim 1, wherein said alcohol is a $C_1$-$C_{18}$ monohydric alcohol, $C_2$-$C_{18}$ diol or polyhydric alcohol.

**5.** The process of claim 4, wherein said alcohol is pentaerythritol or glycerol.

**6.** The process of claim 1, wherein said blend is added to the acid and alcohol reactants prior to the initiation of the esterification reaction.

**7.** The process of claim 1, wherein said phosphonate and said sulfide are present in a weight ratio ranging from 10:1 to 1:20 and preferably from 2:1 to 1:1.

**8.** The process of claim 1, wherein each hydroxy group is in the ortho- or para-position relative to the disulfide linkage, n is 2, m is 2 and each R is positioned meta to the disulfide linkage.

**9.** The process of claim 1, wherein R is straight-chain or branched alkyl with 1 to 12 carbon atoms.

**10.** The process of claim 1, wherein R is methyl or tert.butyl.

**11.** The process of claim 8, wherein said disulfide is selected from the group consisting of 4,4′ dithiobis-(2,6-di-tert.butylphenol), 4,4′-dithiobis(2-methyl-6-tert.butylphenol), 2,2′-dithiobis(4,6-di-tert.butylphenol) and a mixed polysulfide of p-tert.amylphenol.

**12.** The process of claim 1, wherein said disulfide is 4,4′-dithiobis(2,6-di-tert.butylphenol).

**13.** The process of claim 11, wherein said disulfide is mixed with minor amounts of the monosulfide and trisulfide equivalents thereof.

**14.** The process of claim 1, wherein a rosin acid is reacted with a 5-30% excess equivalent amount of pentaerythritol in the presence of 0.2-1.5%, by weight, of said blend.

## Revendications

1. Dans un procédé d'estérification selon lequel on fait réagir un acide carboxylique aliphatique ou aromatique ou un anhydride d'un tel acide avec une quantité équivalente, ou un excès d'au plus 30 %, d'un alcool, à une température élevée, comprise entre 150 et 300°C, en présence d'un catalyseur, pendant un temps suffisant pour qu'on obtienne un ester ayant un indice d'acide d'au plus environ 15, le perfectionnement caractérisé en ce qu'on effectue ladite réaction en présence de 0,05 à 10,0 % en poids d'un mélange de bis[monoéthyl(3,5-di-tert.butyl-4-hydroxybenzyl)-phosphonate] de calcium et d'un sulfure de phénol répondant à la formule

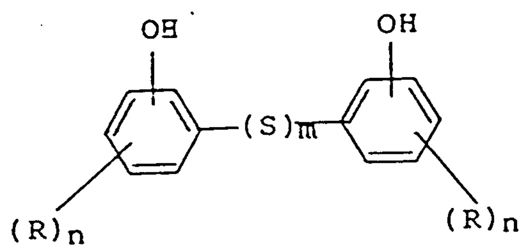

dans laquelle R représente un alkyle en $C_1$-$C_{18}$, un cycloalkyle en $C_5$-$C_{12}$, un aralkyle en $C_7$-$C_9$ ou un aralkyle en $C_7$-$C_9$ portant un alkyle en $C_1$-$C_{12}$, n désigne un nombre de 1 à 3 et m un nombre de 1 à 3.

2. Procédé selon la revendication 1 dans lequel l'acide est un acide monocarboxylique aliphatique en $C_1$-$C_{18}$, un acide dicarboxylique aliphatique en $C_2$-$C_{18}$, un acide tricarboxylique aliphatique, un acide mono-, di- ou tri-carboxylique aromatique, ou un acide résinique.

3. Procédé selon la revendication 2 dans lequel l'acide est un acide résinique.

4. Procédé selon la revendication 1 dans lequel l'alcool est un monoalcool en $C_1$-$C_{18}$, un diol en $C_2$-$C_{18}$ ou un polyalcool.

5. Procédé selon la revendication 4 dans lequel l'alcool est le pentaérythritol ou le glycérol.

6. Procédé selon la revendication 1 dans lequel ledit mélange est ajouté aux composantes acides et alcools avant le début de la réaction d'estérification.

7. Procédé selon la revendication 1 dans lequel le phosphonate et le sulfure sont présents dans un rapport pondéral compris entre 10:1 et 1:20, de préférence entre 2:1 et 1:1.

8. Procédé selon la revendication 1 dans lequel chacun des radicaux hydroxy est en position ortho ou para par rapport à la liaison disulfure, n est égal à 2, m est égal à 2 et chacun des radicaux R est en position méta par rapport à la liaison disulfure.

9. Procédé selon la revendication 1 dans lequel R est un alkyle linéaire ou ramifié, contenant de 1 à 12 atomes de carbone.

10. Procédé selon la revendication 1 dans lequel R est un radical méthyle ou tert-butyle.

11. Procédé selon la revendication 8 dans lequel ledit disulfure est pris dans l'ensemble constitué par le 4,4'-dithio-bis(2,6-di-tert-butylphénol), le 4,4'-dithio-bis(2-méthyl-6-tert-butylphénol), le 2,2'-dithio-bis(4,6-di-tert-butylphénol) et un polysulfure mixte du p-tert-pentyl-phénol.

12. Procédé selon la revendication 1 dans lequel le disulfure est le 4,4'-dithio-bis(2,6-di-tert-butylphénol).

13. Procédé selon la revendication 11 dans lequel le disulfure est mélangé avec de faibles quantités des équivalents monosulfure et trisulfure.

14. Procédé selon la revendication 1 dans lequel on fait réagir un acide résinique avec une quantité de pentaérythritol représentant un excès de 5 à 30 % par rapport à la quantité équivalente, en présence de 0,2 à 1,5 %, en poids dudit mélange.

## Patentansprüche

1. Verfahren zur Veresterung von aliphatischen oder aromatischen Carbonsäuren oder -anhydriden, worin die Carbonsäure oder das -anhydrid mit einer äqiuvalenten Menge oder maximal 30 %igem Ueberschuss eines Alkohols bei erhöhter Temperatur von 150 bis 300 °C in der Gegenwart eines Katalysators solange umgesetzt wird, bis ein Ester mit einer Säurezahl von maximal 15 erhalten wird, dadurch gekennzeichnet, dass die Reaktion in Gegenwart von 0,05-10,0 Gew.-% einer Mischung aus Kalzium-bis[monoethyl(3,5-di-tert.-butyl-4-hydroxybenzyl)-phosphonat und eines phenolischen Sulfides der Formel

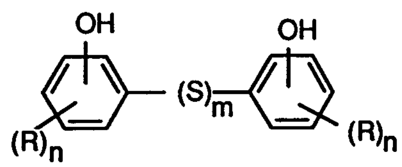

worin R $C_1$-$C_{18}$-Alkyl, $C_5$-$C_{12}$-Cycloalkyl oder unsubstituiertes oder mit $C_1$-$C_{12}$-Alkyl substituiertes $C_7$-$C_9$-Aralkyl bedeutet, n eine Zahl von 1 bis 3 darstellt und m für eine Zahl von 1 bis 3 steht, durchgeführt wird.

2. Verfahren nach Anspruch 1, worin die Säure eine aliphatische Mono-Carbonsäure mit 1 bis 18 C-Atomen, eine aliphatische Bi-Carbonsäure mit 2 bis 18 C-Atomen, eine aliphatische Tri-Carbonsäure, eine aromatische Mono-, Di- oder Tri-Carbonsäure oder eine Harzsäure ist.

3. Verfahren nach Anspruch 2, worin die Säure eine Harzsäure ist.

4. Verfahren nach Anspruch 1, worin der Alkohol ein einwertiger $C_1$-$C_{18}$-Alkohol, ein $C_2$-$C_{18}$-Diol, oder ein mehrwertiger Alkohol ist.

5. Verfahren nach Anspruch 4, worin der Alkohol Pentaerythritol oder Gycerin ist.

6. Verfahren nach Anspruch 1, worin die Mischung vor Beginn der Reaktion zu den Säure- und Alkohol-Reaktanden gegeben wird.

7. Verfahren nach Anspruch 1, worin das Phosphonat und das Sulfid in einem Gewichtsverhältnis von 10:1 bis 1:20 und bevorzugt von 2:1 bis 1:1, zugegen sind.

8. Verfahren nach Anspruch 1, worin jede Hydroxylgruppe in der orth- oder para-Position zur Disulfid-Bindung steht, n 2 ist, m 2 ist und jedes R meta zur Disulfid-Bindung steht.

9. Verfahren nach Anspruch 1, worin R ein geradkettiges oder verzweigtes Alkyl mit 1 bis 12 C-Atomen darstellt.

10. Verfahren nach Anspruch 1, worin R Methyl oder tert.-Butyl bedeutet.

11. Verfahren nach Anspruch 8, worin das Disulfid 4,4'-Dithiobis(2,6-di-tert.-butylphenol),4,4'-Dithiobis(2-methyl-6-tert.-butylphenol), 2,2'-Dithiobis(4,6-di-tert.-butylphenol oder ein gemischtes Polysulfid von p-tert-Amylphenol ist.

12. Verfahren nach Anspruch 1, worin das Disulfid 4,4'-Dithiobis(2,6-di-tert.-butylphenol)ist.

13. Verfahren nach Anspruch 11, worin das Disulfid mit geringen Anteilen der dazu äquivalenten Mono- und Trisulfide gemischt ist.

**14.** Verfahren nach Anspruch 1, worin eine Harzsäure mit einem 5-30 %igen Ueberschuss von Pentaerythritol in der Gegenwart von 0,2-1,5 Gew.-% der Mischung umgesetzt wird.